**Europäisches Patentamt**

**European Patent Office**

**Office européen des brevets**

(19)

(11) Veröffentlichungsnummer : **0 063 339**
**B1**

(12) **EUROPÄISCHE PATENTSCHRIFT**

(45) Veröffentlichungstag der Patentschrift :
**28.08.85**

(21) Anmeldenummer : **82103076.4**

(22) Anmeldetag : **09.04.82**

(51) Int. Cl.⁴ : **A 61 M   5/30**

(54) **Kolbenpumpe für nadellose Injektionsgeräte.**

(30) Priorität : **16.04.81 DE 3115372**

(43) Veröffentlichungstag der Anmeldung :
**27.10.82 Patentblatt 82/43**

(45) Bekanntmachung des Hinweises auf die Patenterteilung : **28.08.85 Patentblatt 85/35**

(84) Benannte Vertragsstaaten :
**CH DE FR GB LI**

(56) Entgegenhaltungen :
**US-A- 3 292 622**
**US-A- 3 833 030**
**US-A- 3 908 651**

(73) Patentinhaber : **HOECHST AKTIENGESELLSCHAFT**
**Postfach 80 03 20**
**D-6230 Frankfurt am Main 80 (DE)**

(72) Erfinder : **Dettbarn, Hans-Jürgen**
**Rehbocks Ecke 4**
**D-3550 Marburg/Lahn (DE)**
Erfinder : **Zimmermann, Josef**
**Auf der Krautweide 11**
**D-6231 Sulzbach (DE)**

## Beschreibung

Die Erfindung betrifft eine Kolbenpumpe für nadellose Injektionsgeräte, bei der das Pumpengehäuse eine Halterung für einen Behälter für das zu injizierende Medium aufweist, die aus einem mit Hohlnadel zur Aufnahme des Behälters versehenen Stutzen, der Teil des Pumpengehäuses ist, und einem Verbindungselement zum Verbinden des Behälters mit dem Stutzen besteht, und das Pumpengehäuse einen Einlaßkanal aufweist, der mit einem Kanal im Stutzen in Verbindung steht.

Kobenpumpen der genannten Art sind aus der deutschen Auslegeschrift 14 91 833 und der US-Patentschrift 3 526 225 bekannt.

Nach der deutschen Auslegeschrift 14 91 833 ist am Gehäuse der Impfstoffpumpe eine Lagerplatte befestigt, die ein Medikamenten- und ein Luftröhrchen sowie den Medikamentenbehälter trägt. Festgeklemmt wird der Medikamentenbehälter durch ein aufspannbares Widerlager — eine Art Bügel —, das aus einem Stopfen, einer Konsole und teleskopartig angeordneten Rohren besteht, die an der Lagerplatte befestigt sind. Diese Konstruktion ist umständlich in der Handhabung. Behälter mit unterschiedlichem Durchmesser sind nur begrenzt verwendbar. Ferner erhöht diese Art der Halterung für den Behälter das Gewicht der Kolbenpumpe und damit das des Injektionsgerätes erheblich.

Nach der US-Patentschrift 3 526 225 wird der Behälter für den Impfstoff durch eine federnde Halterung aus Draht auf dem Nadelstutzen gehalten. Nachteilig bei dieser Art der Halterung ist, daß der Behälter zwar gegen Taumeln durch die Drahtbügel gesichert wird, jedoch nicht gegen Herausrutschen. Auch der Nadelstutzen weist keinerlei Elemente auf, die ein Auseinandergleiten der Verbindung Behälter Nadelstutzen verhindern würde. Ferner sind auch hier Behälter mit unterschiedlichem Durchmesser nur begrenzt verwendbar.

Hier will die Erfindung Abhilfe schaffen. Die Erfindung wie sie in den Ansprüchen gekennzeichnet ist, löst die Aufgabe dadurch, daß das Verbindungselement aus einem U-förmig gebogenen Blech besteht, das auf seiner Innenseite mit U-förmigen Jochen versehen ist, wobei das dem Pumpengehäuse zugewandte Joch in eine im Stutzen vorgesehene Nut eingreift und das andere Joch zum Umfassen des Behälterhalses dient.

Der durch die Erfindung erreichte Vorteil ist im wesentlichen darin zu sehen, daß der Behälter für das zu injizierende Medium auf einfache Weise starr mit der Kolbenpumpe verbunden werden kann. Diese Art der Verbindung ist für alle handelsüblichen Behälter mit der erfindungsgemäßen Halterung möglich.

Im folgenden wird die Erfindung anhand einer lediglich einen Ausführungsweg darstellenden Zeichnung näher erläutert, die eine Seitenansicht der Kolbenpumpe mit aufgestecktem Behälter teilweise geschnitten zeigt.

Das Pumpengehäuse (1) der Kolbenpumpe A für nadellose Injektionsgeräte trägt eine Halterung E für einen Behälter (3), in dem sich das zu injizierende Medium befindet. Die Halterung E besteht aus dem Stutzen (4) und einem Verbindungselement, das den Behälter (3) mit dem Stutzen (4) verbindet. Der Stutzen (4), der Teil des Pumpengehäuses ist, trägt eine Hohlnadel (5). Das zu injizierende Medium gelangt über die Hohlnadel (5), Kanal (6) im Stutzen (4) und Einlaßkanal (7) im Pumpengehäuse (1) vom Behälter (3) in die Pumpenkammer (8). Das Verbindungselement besteht aus einem U-förmig gebogenen Blech (9), das auf seiner Innenseite mit U-förmigen Jochen (10) und (11) versehen ist. Das Joch (10) greift in die Nut (12) des Stutzens (4) ein, das Joch (11) umfaßt den Behälterhals. Der Abstand der Joche (10) und (11) ist so bemessen, daß nach dem Aufstecken des Behälters (3) auf die Hohlnadel (5) durch die Spannkraft des Verschlußstopfens (15) des Behälters (3) der Stutzen (4) und der Behälter (3) über Blech (9) gegeneinander verspannt sind. Durch Aufstecken eines geeigneten Adapters (nicht dargestellt) auf das obere Joch (11) kann das Blech (9) an Behälter mit verschiedenen Halsgrößen angepaßt werden.

In der Hohlnadel (5) ist ein Belüftungsröhrchen (13) angeordnet. Dieses wird durch eine seitliche Öffnung (14) in die Hohlnadel (5) eingeführt. Durch die seitliche Öffnung (14) der Hohlnadel (5) gelanagt das zu injizierende Medium in die Hohlnadel.

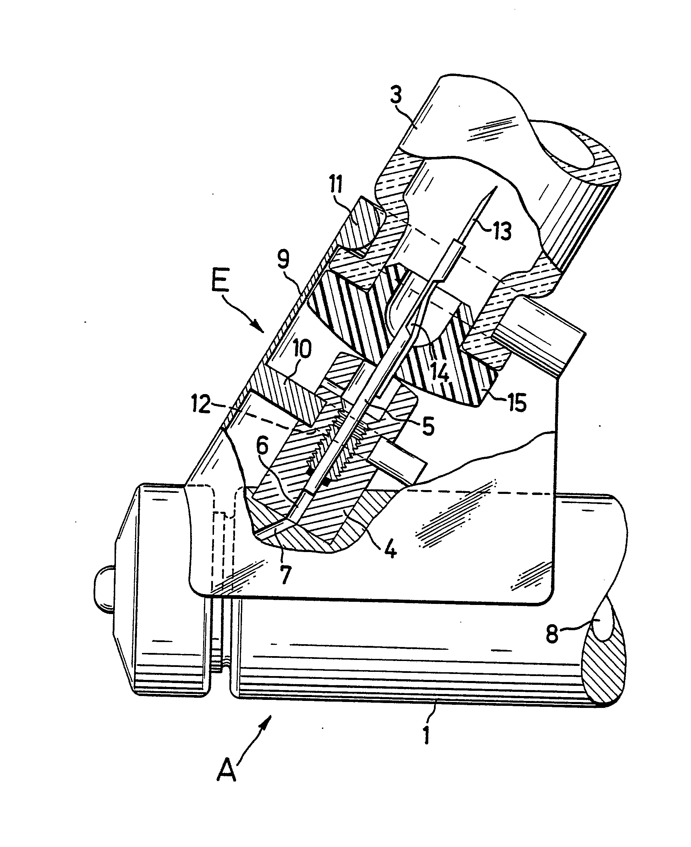

## Patentansprüche

1. Kolbenpumpe für nadellose Injektionsgeräte, bei der das Pumpengehäuse (1) eine Halterung (E) für einen Behälter (3) für das zu injizierende Medium aufweist, die aus einem mit Hohlnadel (5) zum Aufnehmen des Behälters (3) versehenen Stutzen (4), der Teil des Pumpengehäuses (1) ist, und einem Verbindungselement zum Verbinden des Behälters (3) mit dem Stutzen (4) besteht, und das Pumpengehäuse (1) einen Einlaßkanal (7) aufweist, der mit einem Kanal (6) im Stutzen (4) in Verbindung steht, dadurch gekennzeichnet, daß das Verbindungselement aus einem U-förmig gebogenen Blech (9) besteht, das auf seiner Innenseite mit U-förmigen Jochen (10, 11) versehen ist, wobei das dem Pumpengehäuse (1) zugewandte Joch (10) in eine im Stutzen (4) vorgesehene Nut (12) eingreift und das andere Joch (11) zum Umfassen des Behälterhalses dient.

2. Kolbenpumpe nach Anspruch 1, dadurch gekennzeichnet, daß in der Hohlnadel (5) ein Belüftungsröhrchen (13) angeordnet ist.

## Claims

1. A piston pump for needle-less injection instruments, in which the pump housing (1) has a mounting (E) for a vessel (3) for the medium to be injected, this mounting consisting of a pipe (4) end which is provided with a hollow needle (5) for receiving the vessel (3) and which is part of the pump housing (1) and of a connecting element for connecting the vessel (3) to the pipe end (4), the pump housing (1) having an inlet channel (7) connected to a channel (6) in the pipe end (4), wherein the connecting element consists of a metal sheet (9) bent in the form of a U, which is provided on its inner side with U-shaped yokes (10, 11) the yoke (10) facing the pump housing (1) engaging into a groove (12) provided in the pipe end (4) and the other yoke (11) serving for surrounding the vessel neck.

2. The piston pump as claimed in claim 1, wherein a venting tube (13) is located in the hollow needle (5).

## Revendications

1. Pompe à piston pour injecteurs sans aiguille, dont le corps de pompe (1) présente un support (E) pour un récipient (3) de la substance à injecter, qui est constitué d'une tubulure (4) faisant partie du corps de pompe (1) et pourvue d'une aiguille creuse (5), sur laquelle doit être fiché le récipient (3), et d'un élément d'assemblage pour relier le récipient (3) à la tubulure (4), et dont le corps de pompe (1) présente un canal d'admission (7) qui communique avec un canal (6) dans la tubulure (4), caractérisée en ce que l'élément d'assemblage est une tôle (9) courbée en U dont le côté intérieur est pourvu de nervures (10, 11) en U, dont l'une (10), dirigée vers le corps de pompe (1), pénètre dans une gorge (12) ménagée dans la tubulure (4) et dont l'autre (11) est destinée à enserrer le goulot du récipient.

2. Pompe à piston selon la revendication 1, caractérisée en ce qu'une canule d'entrée d'air (13) est disposée dans l'aiguille creuse (5).